# EUROPÄISCHE PATENTSCHRIFT

(11) **EP 2 229 066 B1**
(45) Veröffentlichungstag und Bekanntmachung des Hinweises auf die Patenterteilung: **10.08.2016**
(21) Anmeldenummer: 08866726.6
(22) Anmeldetag: 08.12.2008
(51) Int. Cl.: A43B 7/24, A43B 3/00, A61B 5/103, A61B 5/00

(54) **VERFAHREN ZUM BEEINFLUSSEN DES PRONATIONSVERHALTENS EINES SCHUHS**
METHOD FOR INFLUENCING THE PRONATION BEHAVIOUR OF A SHOE
PROCÉDÉ POUR INFLUER SUR LE COMPORTEMENT D'UNE CHAUSSURE EN MATIÈRE DE PRONATION

(30) Priorität: 29.12.2007 DE 102007063160
(43) Veröffentlichungstag der Anmeldung: 22.09.2010
(73) Patentinhaber: PUMA SE, 91074 Herzogenaurach (DE)
(72) Erfinder: MILANI, Thomas, 04416 Markkleeberg (DE); STERZING, Thorsten, 09113 Chemnitz (DE); ODENWALD, Stephan, 09125 Chemnitz (DE); DÖRFLER, Ralph, 09114 Chemnitz (DE)
(74) Vertreter: Gosdin, Michael
(86) Internationale Anmeldenummer: PCT/EP2008/010374
(87) Internationale Veröffentlichungsnummer: WO 2009/083099

(56) Entgegenhaltungen:
- US-A- 5 813 142
- US-A1- 2002 040 601
- US-A1- 2003 009 308
- US-B1- 6 836 744

## Beschreibung

Die Erfindung betrifft ein Verfahren zum Beeinflussen des Pronationsverhaltens eines Schuhs, insbesondere eines Sportschuhs, bei dem
a) mindestens ein für das Pronationsverhalten relevanter Parameter gemessen wird,
b) der gemessene Parameter einer Steuereinheit zugeleitet wird,
c) die Steuereinheit ein das Pronationsverhalten beeinflussendes Stellsignal an ein Stellelement ausgibt und
d) das Stellelement eine für das Pronationsverhalten relevante Eigenschaft des Schuhs verändert.

Es sind im Stand der Technik Systeme bekannt, die in Schuhen, insbesondere in Sportschuhen, integriert sind, mit denen aktiv eine Eigenschaft des Schuhs beeinflusst werden kann, um die Pronation zu beeinflussen. Diese Systeme werden mit dem gattungsgemäßen Verfahren betrieben. Beispielsweise kann das Feder- bzw. Dämpfungsverhalten des Schuhs beeinflusst werden. Hierfür werden bestimmte Bewegungszustände des Schuhs gemessen und mittels beeinflussbarer Elemente die Feder- bzw. Dämpfungseigenschaften des Schuhs gezielt eingestellt. Eine Lösung dieser Art ist beispielsweise in der US 5 813 142 A offenbart. Ähnliche und andere Lösungen sind in der US 2002/040601 A1, in der US 6 836 744 B1 und in der US 2003/0009308 A1 beschrieben.

In dem erstgenannten Dokument ist ein Sensorsystem zur Ermittlung des Drucks in einer Kammer vorgesehen, die fluidbeaufschlagbar in der Schuhsohle integriert ist. Abhängig vom gemessenen Druck veranlasst eine Steuereinrichtung die Medienzufuhr in die Fluidkammer.

Mit einem solchen System kann grundsätzlich auch - was sehr wünschenswert ist - auf die pronationsregulierende Wirkung des Schuhs beim Aufsetzen desselben auf den Boden Einfluss genommen werden.

Die Pronation ist eine Drehung des Fußes um die Achse des unteren Sprunggelenks, bei der der äußere Fußrand gehoben und der innere Fußrand gesenkt wird. Die Pronation wird auch als Einwärtsdrehung oder Einwärtskantung bezeichnet.

Die normale Pronation des Fußes ist ein natürlicher Dämpfungsmechanismus und eine natürliche Bewegung nach innen beim Fußaufsatz. Allerdings knickt der Fußrand bei der sog. Überpronation stark nach innen ein und belastet damit die Bänder, Sehnen und Gelenke. Diese Überpronation kann verschiedenste Ursachen haben, wie zum Beispiel eine Fußfehlstellung, Übergewicht oder starke Ermüdung. Ebenfalls tritt die Überpronation gelegentlich bei Laufanfängern auf, da der Stützapparat des Fußes noch nicht ausreichend trainiert ist. An den Schuhen ist dann eine starke Abnutzung im medialen Bereich erkennbar.

Der der Pronation entgegengesetzte Mechanismus (auch Supination genannt) tritt beim Laufen seltener auf. Bei der Supination geht die Belastung in die entgegengesetzte Richtung. Bei Laufschuhen ist dies durch eine höhere Abnutzung im lateralen Bereich (also an der Außenseite) erkennbar.

Demgemäß wird es bei modernen Sportschuhen angestrebt, über die an sich bekannten in die Sohle eingearbeiteten Pronationsstützen hinaus eine aktive Beeinflussung der Pronation vorzunehmen, was mit einem System, wie es in der genannten Schrift erläutert ist, möglich ist.

Die bekannten Systeme werden dabei bei Bedarf eingeschaltet und so lange kontinuierlich betrieben, wie es erforderlich ist, d. h. über die gesamte Dauer des Einsatzes des Schuhs. Demgemäß werden über die gesamte Einschaltzeit hinweg Messdaten erfasst, in einem Prozessor verarbeitet und auf ein Stellelement eingewirkt. Das System ist also auch dann aktiv, wenn der Fuß während des Laufens gar nicht den Boden berührt.

Dabei stellt es ein Problem dar, die tatsächlich für die Pronationsbeeinflussung relevanten Daten zu messen bzw. zu bestimmen, mit denen effektiv auf die Pronation Einfluss genommen werden kann. Diese Daten sind nicht in einfacher Weise von den sonst die Laufdynamik bestimmenden Parametern zu isolieren.

Der Erfindung liegt daher die Aufgabe zugrunde, ein Verfahren der eingangs genannten Art so fortzubilden, dass es möglich wird, in verbesserter und einfacherer Weise die relevanten Daten zu bestimmen, die für die Pronationsmessung wesentlich sind. Die Datenbestimmung und die Beeinflussung der Pronation soll so auf eine verbesserte Referenz zurückgeführt werden, um dadurch eine verbesserte Pronationsregelung zu bewerkstelligen. Dabei soll dies in besonders einfacher Weise ermöglicht werden.

Die Lösung dieser Aufgabe durch die Erfindung ist dadurch gekennzeichnet, dass die Verarbeitung von gemäß obigem Schritt a) gemessenen Werten eines Parameters von einem Referenzzeitpunkt ab erfolgt, wobei dieser vom Aufsetzen des Schuhs auf dem Boden bestimmt wird, wobei das Aufsetzen des Schuhs auf dem Boden mit einem Sensor ermittelt wird, der in den Schuh integriert ist und der beim Aufsetzen des Schuhs auf dem Boden an die Steuereinheit ein für das Aufsetzen charakterisierendes Signal ausgibt, wobei die Verarbeitung von gemäß Schritt a) gemessenen Werten eines Parameters beendet wird, wenn das Abheben des Schuhs vom Boden detektiert wird oder die Verarbeitung von gemäß Schritt a) gemessenen Werten eines Parameters beendet wird, wenn eine vorbestimmte Zeit ab dem Referenzzeitpunkt verstrichen ist.

Der Referenzzeitpunkt kann dabei der Kontaktzeitpunkt des Schuhs mit dem Boden selber sein oder von diesem abhängen.

Als Sensor kann ein Druck- oder Kraftsensor verwendet werden, der den vom Fuß des Trägers des Schuhs auf die Sohle des Schuhs ausgeübten Druck bzw. die ausgeübte Kraft misst.

Alternativ kann als Sensor auch ein Beschleunigungssensor verwendet werden, der die Beschleunigung bzw. Verzögerung des Schuhs beim Auftreffen des Schuhs auf dem Boden messen kann.

Gemäß einer weiteren Alternative kann der Sensor auch ein Wegsensor sein, der die Sohlendeformation beim Aufsetzen des Schuhs auf dem Boden misst, wodurch der beginnende Bodenkontakt festgestellt werden kann.

Der Sensor ist bevorzugt im Fersenbereich des Schuhs angeordnet. Er kann in der Sohle zwischen der Unterseite des Fußes des Trägers und der Bodenkontaktfläche der Sohle angeordnet werden. Der Sensor kann insbesondere zwischen einer Außensohle und einer Mittelsohle des Schuhs angeordnet sein.

Die Verarbeitung von gemäß obigem Schritt a) gemessenen Werten eines Parameters wird also beendet, wenn das Abheben des Schuhs vom Boden detektiert wird. Demgemäß erfolgt also eine Verwertung der gemessenen Daten im wesentlichen über den Zeitraum, in dem der Schuh Kontakt mit dem Boden hat.

Alternativ ist es aber auch möglich, dass die Verarbeitung von gemäß obigem Schritt a) gemessenen Werten eines Parameters dann beendet wird, wenn eine vorbestimmte Zeit ab dem Referenzzeitpunkt verstrichen ist. Es kann hier beispielsweise vorgesehen werden, dass eine Verwertung der Daten im oben erläuterten Verfahren ab dem Kontaktzeitpunkt des Schuhs mit dem Boden für 250 ms erfolgt und anschließend die Verarbeitung der Daten beendet wird.

Nach dem Ende der Verarbeitung von gemäß obigem Schritt a) gemessenen Werten eines Parameters kann das Stellelement in eine Referenz- oder Null-Lage oder generell in eine geeignete bzw. berechnete Lage gefahren werden.

Wenngleich die Verarbeitung gemessener Werte erfindungsgemäß nicht ständig erfolgt, kann dennoch vorgesehen werden, dass die Messung der für das Pronationsverhalten relevanter Parameter gemäß obigem Schritt a) ständig erfolgt, insbesondere also auch während der Phasen, in denen kein Kontakt des Schuhs mit dem Boden vorliegt. Es wird dann also beim permanenten Einlauf von Messwerten auf diese zwecks Verwertung nur in gewissen Zeitintervallen zugegriffen.

In diesem Falle kann gemäß einer Fortbildung der Erfindung vorgesehen werden, dass die Steuereinheit die Steuersignale an das Stellelement unter Berücksichtigung von gemessenen Parameterdaten abgibt, die bereits während eines definierten Zeitraums vor dem Aufsetzen des Schuhs auf dem Boden gemessen wurden. Wenn also die Messwerte ständig erhoben werden, kann beispielsweise vorgesehen werden, die bereits im Zeitintervall von 10 ms vor dem Aufsetzen des Schuhs auf dem Boden gemessenen Werte bei der Pronationsregelung einzubeziehen.

Ein für das Pronationsverhalten relevanter Parameter kann die Drehung oder die Drehgeschwindigkeit des Schuhs um eine vorgegebene Achse sein.

Die Messung der Drehung oder der Drehgeschwindigkeit des Schuhs um die Achse kann dabei mit einem am oder im Schuh angeordneten Kreiselmesssystem erfolgen.

Die Achse weist dabei in ihrer Projektion auf die Bodenfläche zu einer Längsachse des Schuhs vorzugsweise einen Winkel zwischen 0° und 45° auf, insbesondere zwischen 0° und 10°. Die Achse kann indes in Richtung der Längsachse gesehen zur Bodenfläche einen Winkel zwischen 0° und 70° aufweisen, insbesondere zwischen 0° und 10°.

Die für das Pronationsverhalten relevante Eigenschaft des Schuhs kann die Dicke der Sohle zwischen der Unterseite des Fußes des Trägers und der Bodenkontaktfläche der Sohle in einem definierten Bereich der Breite des Schuhs quer zur Längsachse des Schuhs sein. Es ist aber auch möglich, dass die für das Pronationsverhalten relevante Eigenschaft des Schuhs die Federsteifigkeit der Sohle in einem definierten Bereich der Breite des Schuhs quer zur Längsachse des Schuhs ist. Es ist weiter möglich, dass die für das Pronationsverhalten relevante Eigenschaft des Schuhs die Winkelstellung zwischen der Ober- und der Unterseite der Sohle ist.

Mit der vorgeschlagenen Ausgestaltung eines Schuhs bzw. der genannten Verfahrensweise zur Beeinflussung der Pronation kann erreicht werden, dass in verbesserter Weise auf diejenigen Messwerte zurückgegriffen werden kann, die besonders relevant für die Pronationsbeeinflussung sind. Abgestellt wird auf den Zeitpunkt, zu dem Bodenkontakt zwischen Schuh und Boden vorliegt, wobei dann für die Zeit des Bodenkontakts oder für eine definierte Zeit die ständig gemessenen Parameterwerte im Rahmen der Pronationsregelung verwertet werden.

Ferner liegt eine energiesparendere Betriebsweise vor, wenn die Regelung der Pronation nur in bestimmten Phasen eines Schrittzyklus erfolgt.

Der Zeitpunkt der Kontaktnahme des Schuhs mit dem Boden dient also als Auslöser für die Beeinflussung der Pronation im erläuterten Sinne. Das Signal der Kontaktnahme kann dabei beispielsweise das Pronationsregelsystem aktivieren, wobei allerdings die Parametermessung ständig erfolgt.

In der Zeichnung ist ein Ausführungsbeispiel der Erfindung dargestellt. Es zeigen:
- Fig. 1: schematisch das Bein eines Läufers mit einem Schuh, kurz vor dem Aufsetzen des Schuhs auf dem Boden,
- Fig. 2a: den Verlauf des Messwerts eines Sensors zur Detektion des Aufsetzens des Schuhs auf dem Boden über der Zeit und
- Fig. 2b: den zugehörigen Verlauf der Aktivität, d. h. des Betriebs, eines Systems zur Beeinflussung der Pronation über der Zeit.

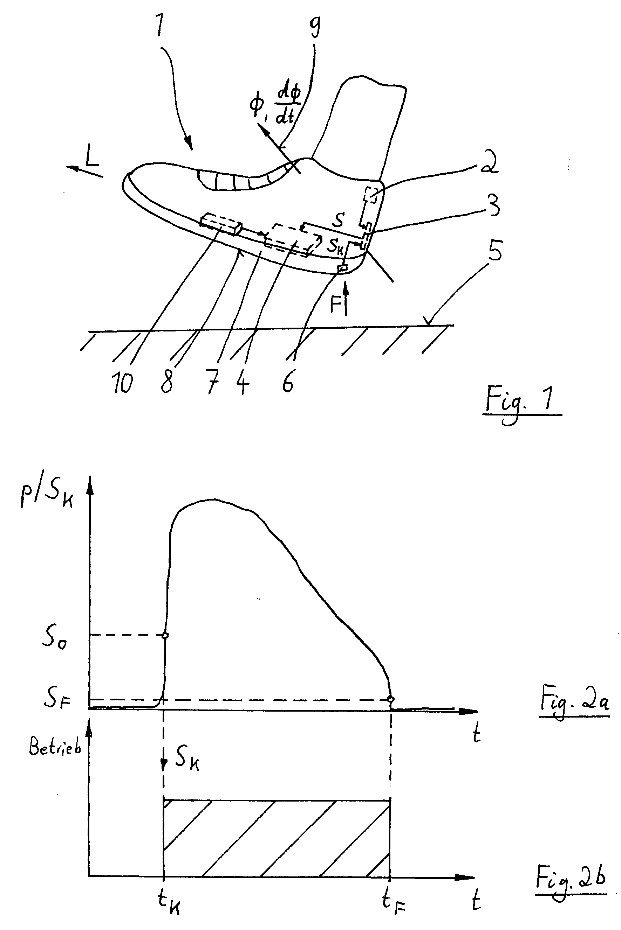

In Fig. 1 ist der untere Bereich des Beins eines Läufers zu sehen, der einen Schuh 1 trägt. Während eines Schritts bzw. während eines Schrittzyklus befindet sich der Schuh 1 über eine gewisse Zeit in der Luft. Dann setzt er auf dem Boden 5 auf und rollt auf diesem ab, bevor er wieder abhebt. Der Schuh 1 hat eine Sohle 7 mit einer Bodenkontaktfläche 8. Dargestellt ist die Situation kurz vor dem Aufsetzen des Schuhs 1 auf dem Boden 5, d. h. der Schuh 1 befindet sich hier noch in der Flugphase.

Der Schuh 1 ist mit einem System 2, 3, 4, 10 zur Beeinflussung der Pronation des Fußes des Trägers des Schuhs 1 ausgestattet. Dieses System besteht zunächst aus einem Sensor 2, der in der Lage ist, einen für die Pronation relevanten Parameter zu erfassen. Vorliegend kommt die Drehgeschwindigkeit dφ/dt des Schuhs als Ableitung eines Winkels φ als relevanter Parameter zum Einsatz. Gemessen wird die Winkelgeschwindigkeit dφ/dt um eine Achse 9, die unter einem Winkel sowohl zur Längsachse L des Schuhs 1 als auch zur Horizontalen geneigt ist. Es hat sich bewährt, als Sensor 2 ein Kreiselmessgerät (Gyrometer) zu verwenden, das in der Lage ist, ein der Winkelgeschwindigkeit proportionales Signal zu liefern.

Dieses Signal (Messwert) wird vom Sensor 2 zu einer Steuereinheit 3 mit Mikroprozessor geleitet, in dem ein Steuer- bzw. Regelalgorithmus gespeichert ist. In Abhängigkeit des ermittelten Messwerts veranlasst die Steuereinheit 3 unter Zugrundelegung des gespeicherten Algorithmus die Ausgabe eines Stellsignals S an ein Stellelement 4, d. h. an einen Aktuator, der in der Lage ist, einen für die Pronation wesentlichen Parameter des Schuhs 1 so zu verändern, dass gezielt auf die Pronation Einfluss genommen werden kann.

Dabei ist insbesondere an ein Stellelement 4 gedacht, das in einem seitlichen Bereich der Sohle 7 die effektive Dicke der Sohle 7 zwischen der Aufstandsfläche des Fußes auf der Sohle und der Bodenkontaktfläche 8 verändern kann. Demgemäß kippt der Fuß beim Auftreffen auf dem Boden bzw. beim Abrollen des Fußes auf dem Boden mehr oder weniger um die Längsachse L des Schuhs 1. Generell ist aber auch ein Stellelement denkbar, das auf die Feder- oder Dämpfungseigenschaften des Schuhs Einfluss nimmt, wie es als solches aus dem Stand der Technik bekannt ist.

Das Stellelement 4 erhält dabei von einer Batterie 10 die Energie, um die jeweiligen Verstellbewegungen ausführen zu können.

Wesentlich ist, dass die Verarbeitung von gemessenen Werten des Parameters φ bzw. dφ/dt von einem Referenzzeitpunkt t_{K} ab erfolgt (s. hierzu Fig. 2b), wobei dieser Zeitpunkt vom Aufsetzen des Schuhs 1 auf dem Boden bestimmt wird.

Zur Ermittlung dieses Zeitpunkts t_{K} ist im Schuh 1 und namentlich in dessen Sohle 7, bevorzugt zwischen einer Außen- und einer Zwischensohle, ein Sensor 6 vorgesehen. Dieser Sensor kann als Druck- bzw. Kraftsensor ausgebildet sein, der in der Lage ist, die zwischen dem Fuß des Trägers und dem Boden 5 wirkende Kraft F (oder einen Druck p) zu messen. Während der Flugphase liegt kein Kontakt zwischen Schuh und Boden 5 vor, so dass die vom Sensor 6 gemessene Kraft im wesentlichen Null ist. Trifft der Schuh nach Abschluss der Flugphase indes auf dem Boden 5 auf, registriert der Sensor 6 einen Wert.

Der Messwert des Sensors 6 wird als Kontakt-Signal S_{K} an die Steuereinheit 3 übermittelt. Sobald ein Signalwert festgestellt wird, der über einem vorgegebenen Schwellenwert S₀ liegt, werden die vom Sensor 2 gemessenen Daten verwertet, um mittels des Systems 2, 3, 4, 10 die Pronation aktiv zu beeinflussen.

Dies ist in Fig. 2a und Fig. 2b illustriert. In Fig. 2a ist der Verlauf des Messwerts des Sensors 6 als Funktion über der Zeit dargestellt. Auf der Ordinate ist also (qualitativ) die Größe des Sensorsignals aufgetragen. Sobald die gemessene Kraft bzw. der gemessene Druck den Schwellenwert S₀ übersteigt, erfolgt die Verwertung der gemessenen Daten. Die Datenmessung selber kann indes ständig erfolgen.

Dies ist in Fig. 2b angedeutet. Zum Zeitpunkt t_{K} liegt das Überschreiten des Schwellenwerts S₀ vor, so dass die Messwertverwertung beginnt (schraffierter Bereich der Messwertverwertung in Fig. 2b). Sinkt der Sensorwert indes wieder auf einen geringen, vorgegebenen Wert S_{F} ab (Beginn der Flugphase), wird auch dies von der Steuereinheit 3 registriert, die daraufhin die Messwertverwertung beendet.

Die Stellbewegungen des Pronationsregelsystems erfolgen indes zumeist während der Flugphase des Schuhs. In dieser Zeit werden erfindungsgemäß gar keine Messdaten von der Steuerung verwertet.

Der Sensor 2 zur Ermittlung eines für die Pronation relevanten Parameters ist hier als Kreiselmesssystem ausgebildet, das in der Lage ist, eine Winkelgeschwindigkeit dφ/dt um die Achse 9 zu messen. Die Achse 9 ist in einer bestimmten Lage im Schuh angeordnet, die für die Determinierung der Pronation des Schuhs bzw. des Fußes des Trägers besonders maßgeblich ist. Sie ist in ihrer Boden-Projektion zur Längsachse L des Schuhs 1 unter einem Winkel angeordnet, der bei ca. 0° bis 10° liegt. Betrachtet man die Achse 9 in Richtung der Längsachse L, schließt sie zur Horizontalen einen Winkel ein, der auch im Bereich von 0° bis 10° liegt. Der Sensor 2 ist im lateralen Bereich des Schuhs 1 angeordnet, und zwar im unteren Bereich des Schuhoberteils.

Der Sensor 6 ist bevorzugt - wie bereits erwähnt - zwischen einer Außen- und einer Mittelsohle des Schuhs 1 angeordnet und zwar direkt am posterioren Rand (d. h. im hinteren Randbereich) des Schuhs 1.

Nicht dargestellt sind gegebenenfalls vorhandene weitere Sensoren 2, wobei beispielsweise an einen im Fersenbereich posterior der Mittelfußbrücke angeordneten Sensor gedacht ist, der als Beschleunigungssensor ausgebildet ist und eine Beschleunigung in Richtung der Horizontalen und quer zur Längsachse L messen kann. Der Beschleunigungssensor misst dann also Horizontalbeschleunigungen in mediolaterale Richtung.

Generell gilt, dass die vorgeschlagene Verfahrensweise zur Beeinflussung des Bewegungsausmaßes und der Bewegungsgeschwindigkeit insbesondere des Schuhfersenbereichs geeignet ist, d. h. um das Ausmaß und die Geschwindigkeit der Pronation zu beeinflussen.

Wie erläutert, kann mit dem Sensor 6 der initiale Bodenkontakt des Schuhs 1 ermittelt werden. Ein Ausschlag des Sensorsignals (zum Zeitpunkt t_{K} in Fig. 2) zeigt, dass der Bodenkontakt des Schuhs und insbesondere der Ferse beginnt. Entsprechend signalisiert ein Druckabfall des Messwerts (zum Zeitpunkt t_{F} in Fig. 2) ein Abheben des Schuhs vom Boden 5.

Um die Drehgeschwindigkeit der Fersenbewegung zu erhalten, wird das Kreiselmesssystem 2 zur Messung der Winkelgeschwindigkeit dφ/dt verwendet. Das ermittelte Maximum entspricht der maximalen Pronationsgeschwindigkeit.

Durch die Integration des vom Kreiselmesssystem 2 gemessenen Signals der Winkelgeschwindigkeit dφ/dt kann das Winkelausmaß der Pronation bestimmt werden. Entsprechend kann durch Integration des Winkelgeschwindigkeitssignals über der Zeit der Verlauf des (Pronations)Winkels bestimmt werden. Als Differenz zwischen dem Minimum und dem Maximum ergibt sich das Pronationsausmaß.

Möglich ist es auch, die Messwert-Verwertung für einen vorgegebenen Zeitraum nach dem Zeitpunkt t_{K} durchzuführen, beispielsweise für einen Zeitraum von 250 ms. Genauso ist es möglich - sofern die Messwerterfassung ständig erfolgt -, bei der Messwertverwertung auf Daten zurückzugreifen, die bereits vor dem Zeitpunkt t_{K} liegen, beispielsweise im Zeitraum von 10 ms vor t_{K}.

Möglich ist es ferner auch, die so gesammelten Messwerte über eine Anzahl von Schritten zu erfassen und dann bei der Pronationsregelung als gemittelte Werte zu berücksichtigen.

Während die vorgeschlagene Vorgehensweise bevorzugt in einem System zur Anwendung kommt, das in den Schuh integriert ist und dort für eine aktive Beeinflussung des Pronationsverhaltens sorgt, ist es grundsätzlich genauso möglich, die vorgeschlagenen Vorgehensweise für stationäre und mobile Messungen zu nutzen, z. B. zur Analyse des Laufverhaltens eines Läufers (z. B. auf einem Laufband).

### Bezugszeichenliste:

- 1: Schuh
- 2: Sensor (Kreiselmesssystem)
- 3: Steuereinheit
- 4: Stellelement
- 5: Boden
- 6: Sensor
- 7: Sohle
- 8: Bodenkontaktfläche
- 9: Achse
- 10: Energiequelle (Batterie)

- S: Stellsignal
- S₀: Schwellenwert
- S_{K}: Signal für das Aufsetzen des Schuhs auf dem Boden
- F: Kraft
- L: Längsachse des Schuhs
- φ, dφ/dt: Parameter
- φ: Winkel
- dφ/dt: Winkelgeschwindigkeit
- t_{K}: Referenzzeitpunkt

## Patentansprüche

1. Verfahren zum Beeinflussen des Pronationsverhaltens eines Schuhs (1), insbesondere eines Sportschuhs, bei dem
a) mindestens ein für das Pronationsverhalten relevanter Parameter (φ, dφ/dt) gemessen (2) wird,
b) der gemessene Parameter (φ, dφ/dt) einer Steuereinheit (3) zugeleitet wird,
c) die Steuereinheit (3) ein das Pronationsverhalten beeinflussendes Stellsignal (S) an ein Stellelement (4) ausgibt und
d) das Stellelement (4) eine für das Pronationsverhalten relevante Eigenschaft des Schuhs (1) verändert,
**dadurch gekennzeichnet,**
**dass** die Verarbeitung von gemäß Schritt a) gemessenen Werten eines Parameters (φ, dφ/dt) von einem Referenzzeitpunkt (t_{K}) ab erfolgt, wobei dieser vom Aufsetzen des Schuhs (1) auf dem Boden bestimmt wird, wobei das Aufsetzen des Schuhs (1) auf dem Boden (5) mit einem Sensor (6) ermittelt wird, der in den Schuh (1) integriert ist und der beim Aufsetzen des Schuhs (1) auf dem Boden (5) an die Steuereinheit (3) ein für das Aufsetzen charakterisierendes Signal (S_{K}) ausgibt,
wobei die Verarbeitung von gemäß Schritt a) gemessenen Werten eines Parameters (φ, dφ/dt) beendet wird, wenn das Abheben des Schuhs (1) vom Boden (5) detektiert wird oder die Verarbeitung von gemäß Schritt a) gemessenen Werten eines Parameters (φ, dφ/dt) beendet wird, wenn eine vorbestimmte Zeit ab dem Referenzzeitpunkt (t_{K}) verstrichen ist.

2. Verfahren nach Anspruch 1, **dadurch gekennzeichnet, dass** als Sensor (6) ein Druck- oder Kraftsensor verwendet wird, der den vom Fuß des Trägers des Schuhs (1) auf die Sohle (7) des Schuhs (1) ausgeübten Druck (p) bzw. die ausgeübte Kraft (F) misst.

3. Verfahren nach Anspruch 1, **dadurch gekennzeichnet, dass** als Sensor (6) ein Beschleunigungssensor verwendet wird, der die Beschleunigung bzw. Verzögerung des Schuhs (1) beim Auftreffen des Schuhs (1) auf dem Boden messen kann.

4. Verfahren nach einem der Ansprüche 1 bis 3, **dadurch gekennzeichnet, dass** der Sensor (6) im Fersenbereich des Schuhs (1) angeordnet wird.

5. Verfahren nach einem der Ansprüche 1 bis 4, **dadurch gekennzeichnet, dass** der Sensor (6) in der Sohle (7) zwischen der Unterseite des Fußes des Trägers und der Bodenkontaktfläche (8) der Sohle (7) angeordnet wird, insbesondere zwischen einer Außensohle und einer Mittelsohle des Schuhs (1).

6. Verfahren nach einem der Ansprüche 1 bis 5, **dadurch gekennzeichnet, dass** nach dem Ende der Verarbeitung von gemäß Schritt a) nach Anspruch 1 gemessenen Werten eines Parameters (φ, dφ/dt) das Stellelement (4) in eine definierte Lage oder in eine Referenz- oder Null-Lage gefahren wird.

7. Verfahren nach einem der Ansprüche 1 bis 6, **dadurch gekennzeichnet, dass** die Messung der für das Pronationsverhalten relevanten Parameter (φ, dφ/dt) gemäß Schritt a) von Anspruch 1 ständig erfolgt, insbesondere auch während der Phasen, in denen kein Kontakt des Schuhs mit dem Boden vorliegt.

8. Verfahren nach Anspruch 7, **dadurch gekennzeichnet, dass** die Steuereinheit (3) die Steuersignale an das Stellelement (4) unter Berücksichtigung von gemessenen Parameterdaten (φ, dφ/dt) abgibt, die bereits während eines definierten Zeitraums vor dem Aufsetzen des Schuhs (1) auf dem Boden gemessen wurden.

9. Verfahren nach einem der Ansprüche 1 bis 8, **dadurch gekennzeichnet, dass** der für das Pronationsverhalten relevante Parameter die Drehung (φ) oder die Drehgeschwindigkeit (dφ/dt) des Schuhs (1) um eine vorgegebene Achse (9) ist.

10. Verfahren nach Anspruch 9, **dadurch gekennzeichnet, dass** die Messung der Drehung (φ) oder der Drehgeschwindigkeit (dφ/dt) des Schuhs (1) um die Achse (9) mit einem am oder im Schuh angeordneten Kreiselmesssystem (2) erfolgt.

11. Verfahren nach Anspruch 9 oder 10, **dadurch gekennzeichnet, dass** die Achse (9) in ihrer Projektion auf die Bodenfläche zu einer Längsachse (L) des Schuhs (1) einen Winkel zwischen 0° und 45°, insbesondere zwischen 0° und 10°, aufweist.

12. Verfahren nach einem der Ansprüche 9 bis 11, **dadurch gekennzeichnet, dass** die Achse (9) in Richtung der Längsachse (L) gesehen zur Bodenfläche einen Winkel zwischen 0° und 70°, insbesondere zwischen 0° und 10°, aufweist.

## Claims

1. Method for influencing the pronation behaviour of a shoe (1), especially of a sports shoe, in which
a) at least on parameter (φ, dφ/dt) is measured (2) which is relevant for the behaviour of pronation,
b) the measured parameter (φ, dφ/dt) is led to a control unit (3),
c) the control unit (3) outputs an actuation signal (S) which is influencing the behaviour of pronation to an actuator (4) and
d) the actuator (4) changes a property of the shoe (1) which property is relevant for the behaviour of pronation,
**characterized in that**
the processing of data of a parameter (φ, dφ/dt) measured according to step a) takes place from a reference point of time (t_{K}), wherein the reference point of time is determined from the touch down of the shoe (1) on the ground, wherein the touch down of the shoe (1) on the ground (5) is detected by a sensor (6) which is integrated into the shoe and which outputs a signal (S_{K}) at the touch down of the shoe (1) on the ground (5) to the control unit (3) which is characteristic for the touch down,
wherein the processing of data of a parameter (φ, dφ/dt) measured according to step a) of claim 1 is terminated when the lift off of the shoe (1) from the ground (5) is detected or that the processing of data of a parameter (φ, dφ/dt) measured according to step a) of claim 1 is terminated when a predetermined time from the reference point of time (t_{K}) is elapsed.

2. Method according to claim 1, **characterized in that** a pressure or force sensor is used as the sensor (6), which measures the pressure (p) or the force (F) respectively which is exerted by the foot of the wearer of the shoe (1) on the sole (7) of the shoe (1).

3. Method according to claim 1, **characterized in that** an acceleration sensor is used as the sensor (6), which can measure the acceleration or deceleration respectively of the shoe (1) during touch down of the shoe (1) on the ground.

4. Method according to one of claims 1 till 3, **characterized in that** the sensor (6) is arranged in the heel region of the shoe (1).

5. Method according to one of claims 1 till 4, **characterized in that** the sensor (6) is arranged in the sole (7) between the bottom side of the foot of the wearer and the ground contact area (8) of the sole (7), especially between an outer sole and a midsole of the shoe (1).

6. Method according to one of claims 1 till 5, **characterized in that** after the end of the processing of data of a parameter (φ, dφ/dt) measured according to step a) of claim 1 the actuator (4) is driven into a defined position or into a reference or zero position.

7. Method according to one of claims 1 till 6, **characterized in that** the measurement of parameters (φ, dφ/dt) which are relevant for the behaviour of pronation according to step a) of claim 1 occurs permanently, especially also during the phases in which no contact exists between the shoe and the ground.

8. Method according to claim 7, **characterized in that** the control unit (3) outputs controlling signals to the actuator (4) incorporating measured data of parameters (φ, dφ/dt) which are already measured within a defined period prior the touch down of the shoe (1) on the ground.

9. Method according to one of claims 1 till 8, **characterized in that** the parameter which is relevant for the behaviour of pronation is the angle (φ) or the angular speed (dφ/dt) of the shoe (1) around a predetermined axis (9).

10. Method according to claim 9, **characterized in that** the measurement of the angle (φ) or the angular speed (dφ/dt) of the shoe (1) around the axis (9) is affected by means of a gyroscopic sensor (2) which is arranged at or in the shoe.

11. Method according to claim 9 or 10, **characterized in that** the projection of the axis (9) on the ground area includes an angle between 0° and 45° with the longitudinal axis (L) of the shoe (1), especially an angle between 0° and 10°.

12. Method according to one of claims 9 till 11, **characterized in that** the axis (9), seen in the direction of the longitudinal axis (L), includes an angle between 0° and 70° with the ground area, especially an angle between 0° and 10°.

## Revendications

1. Procédé pour influencer le comportement de pronation d'une chaussure (1), en particulier d'une chaussure de sport, dans lequel :
a) au moins un paramètre (φ, dφ/dt) pertinent pour le comportement de pronation est mesuré (2),
b) le paramètre mesuré (φ, dφ/dt) est envoyé à une unité de commande (3),
c) l'unité de commande (3) délivre un signal de commande (S) influençant le comportement de pronation à un élément de commande (4), et
d) l'élément de commande (4) modifie une propriété de la chaussure (1) pertinente pour le comportement de pronation,
**caractérisé en ce que**
le traitement de valeurs d'un paramètre (φ, dφ/dt) mesurées à l'étape a) s'effectue à partir d'un instant de référence (t_{K}), celui-ci étant déterminé par la pose de la chaussure (1) sur le sol, la pose de la chaussure (1) sur le sol (5) étant déterminée avec un capteur (6) qui est intégré dans la chaussure (1) et qui, lors de la pose de la chaussure (1) sur le sol (5), envoie à l'unité de commande (3) un signal (S_{K}) caractérisant la pose,
le traitement de valeurs d'un paramètre (φ, dφ/dt) mesurées à l'étape a) se termine lorsque le soulèvement de la chaussure (1) du sol (5) est détecté ou le traitement de valeurs d'un paramètre (φ, dφ/dt) mesurées à l'étape a) se termine lorsqu'un temps prédéterminé à partir de l'instant de référence (t_{K}) s'est écoulé.

2. Procédé selon la revendication 1, **caractérisé en ce que** l'on utilise comme capteur (6) un capteur de pression ou de force qui mesure la pression (p) exercée ou la force (F) exercée par le pied du porteur de la chaussure (1) sur la semelle (7) de la chaussure (1).

3. Procédé selon la revendication 1, **caractérisé en ce que** l'on utilise comme capteur (6) un capteur d'accélération qui peut mesurer l'accélération ou le ralentissement de la chaussure (1) lors de l'impact de la chaussure (1) sur le sol.

4. Procédé selon l'une quelconque des revendications 1 à 3, **caractérisé en ce que** le capteur (6) est disposé dans la zone du talon de la chaussure (1).

5. Procédé selon l'une quelconque des revendications 1 à 4, **caractérisé en ce que** le capteur (6) est disposé dans la semelle (7) entre le côté inférieur du pied du porteur et la surface de contact avec le sol (8) de la semelle (7), en particulier entre une semelle extérieure et une semelle intermédiaire de la chaussure (1).

6. Procédé selon l'une quelconque des revendications 1 à 5, **caractérisé en ce qu'**après la fin du traitement de valeurs d'un paramètre (φ, dφ/dt) mesurées à l'étape a) selon la revendication 1, l'élément de commande (4) est déplacé dans une position définie ou dans une position de référence ou position zéro.

7. Procédé selon l'une quelconque des revendications 1 à 6, **caractérisé en ce que** la mesure des paramètres (φ, dφ/dt) pertinents pour le comportement de pronation selon l'étape a) de la revendication 1 s'effectue en continu, notamment également pendant les phases dans lesquelles aucun contact de la chaussure avec le sol n'a lieu.

8. Procédé selon la revendication 7, **caractérisé en ce que** l'unité de commande (3) délivre les signaux de commande à l'élément de commande (4) en tenant compte de données de paramètres mesurées (φ, dφ/dt) qui ont déjà été mesurées pendant un intervalle défini avant la pose de la chaussure (1) sur le sol.

9. Procédé selon l'une quelconque des revendications 1 à 8, **caractérisé en ce que** le paramètre pertinent pour le comportement de pronation est la rotation (φ) ou la vitesse de rotation (dφ/dt) de la chaussure (1) autour d'un axe (9) prédéfini.

10. Procédé selon la revendication 9, **caractérisé en ce que** la mesure de la rotation (φ) ou de la vitesse de rotation (dφ/dt) de la chaussure (1) autour de l'axe (9) s'effectue avec un système de mesure gyroscopique (2) disposé sur ou dans la chaussure.

11. Procédé selon la revendication 9 ou 10, **caractérisé en ce que** l'axe (9) présente, dans sa projection sur la surface du sol par rapport à un axe longitudinal (L) de la chaussure (1), un angle compris entre 0° ° et 45°, en particulier compris entre 0° et 10°.

12. Procédé selon l'une quelconque des revendications 9 à 11, **caractérisé en ce que** l'axe (9), vu dans la direction de l'axe longitudinal (L) par rapport à la surface du sol, présente un angle compris entre 0° et 70°, en particulier compris entre 0° et 10°.
